# EUROPEAN PATENT APPLICATION

(11) **EP 2 279 740 A1**
(43) Date of publication of application: **02.02.2011**
(21) Application number: 09754777.2
(22) Date of filing: 28.05.2009
(51) Int. Cl.: A61K 31/4465, A61K 9/70, A61P 25/28

(54) **TRANSDERMAL PREPARATION**

(30) Priority: 30.05.2008 JP 2008143591; 30.05.2008 US 129010
(71) Applicant: Eisai R&D Management Co., Ltd., Tokyo 112-8088 (JP); Nitto Denko Corporation, Ibaraki-shi Osaka 567-8680 (JP)
(72) Inventor: HANATANI, Akinori, Ibaraki-shi Osaka 567-8680 (JP); SEKIYA, Junichi, Ibaraki-shi Osaka 567-8680 (JP); SAKAMOTO, Sachiko, Ibaraki-shi Osaka 567-8680 (JP); MARUYAMA, Sumiyo, Ibaraki-shi Osaka 567-8680 (JP); AKEMI, Hitoshi, Ibaraki-shi Osaka 567-8680 (JP)
(74) Representative: HOFFMANN EITLE
(86) International application number: PCT/JP2009/059798
(87) International publication number: WO 2009/145269

(57) **Abstract**

The present invention provides a percutaneously absorbable preparation comprising 2-[(1-benzylpiperidin-4-yl) methyl]-5,6-dimethoxyindan-1-one and/or a hydrochloride thereof wherein the percuneously absorbable preoaration is able to administer to a patient such that Cmax per unit surface area of the percutaneously absorbable preparation is 0.025 to 0.5 ng/ml·cm² in a plasma concentration profile.

## Description

### TECHNICAL FIELD

The present invention relates to a percutaneously absorbable preparation comprising 2-[(1-benzylpiperidin-4-yl)methyl]-5,6-dimethoxyindan-1-one (to be referred to as "donepezil") and/or a hydrochloride thereof in the form of 2-[(1-benzylpiperidin-4-yl)methyl]-5,6-dimethoxyindan-1-one monohydrochloride (to be referred to as "donepezil hydrochloride"). Specifically, the present invention relates to a percutaneously absorbable preparation having a favorable plasma drug concentration profile to be applied as a patch to the skin surface in order to continuously administer this drug to a living body through the skin.

### BACKGROUND ART

The basic drug, donepezil or its hydrochloride, donepezil hydrochloride, has acetylcholine esterase inhibitory action and is used against Alzheimer's dementia. Alzheimer's dementia patients are usually elderly, and often have difficulty swallowing oral dosage. In some cases it may also be difficult to administer oral dosage to patients with advanced symptoms of Alzheimer's dementia. In these cases, non-oral administration is useful.

In addition, in the case of oral administration of donepezil commercially available in the form of a rapid-release tablet, plasma concentrations of the drug in a patient reach a maximum concentration within 2 to 5 hours after administering the drug, and demonstrate a sharp, spike-like curve (Patent document 1).

However, the demonstration of a spike-like curve by the plasma concentration of a drug as the Tmax thereof has the risk of causing adverse side effects accompanying the sudden increase in plasma concentration.

In such cases, preparations generating a gentle increase in plasma drug concentration are useful.

On the other hand, percutaneously absorbable preparations using donepezil or donepezil hydrochloride are already known as parenteral administration forms, and for example a preparation for percutaneous use (such as an ointment or skin patch) comprising donepezil hydrochloride is proposed in Patent document 2. Patent document 3 also proposes a percutaneously absorbable dementia treatment preparation comprising donepezil or its hydrochloride in a pressure-sensitive adhesive composition, and describes that when using a hydrochloride of donepezil, a satisfactory skin permeation rate can be obtained by including an acetic acid salt in the pressure-sensitive adhesive composition
Patent document 1 : US2007/0129402
Patent document 2 : Japanese Patent Application Laid-open No. H11-315016
Patent document 3 : WO 2003/032960

### SUMMARY OF INVENTION

### Problems to be Solved by the Invention

However, a percutaneously absorbable preparation of donepezil that has a more favorable plasma drug concentration profile and has less risk of the occurrence of adverse side effects than donepezil commercially available in the form of rapid-release tablets is not known.

Under these circumstances, it is an object of the present invention to provide a percutaneously absorbable preparation for percutaneous absorption of donepezil and/or donepezil hydrochloride that has a favorable plasma drug concentration profile without the occurrence of a sudden increase in plasma concentration.

### Means for Solving the Problems

As a result of conducting extensive studies to solve the aforementioned problems, the present inventors found that a percutaneously absorbable preparation comprising donepezil and/or donepezil hydrochloride is a percutaneously absorbable preparation demonstrating a favorable plasma drug concentration profile and capable of inhibiting the risk of the occurrence of adverse side effects, thereby leading to completion of the present invention.

Namely, the present invention is as follows:
[1] a percutaneously absorbable preparation comprising 2-[(1-benzylpiperidin-4-yl) methyl]-5,6-dimethoxyindan-1-one and/or a hydrochloride thereof,
   wherein the percutaneously absorbable preparation is able to administer to a patient such that Cmax per unit surface area of the percutaneously absorbable preparation is 0.025 to 0.5 ng/ml·cm² in a plasma concentration profile;
[2] the percutaneously absorbable preparation described in [1] above, wherein the Cmax is 0.025 to 0.45 ng/ml·cm²;
[3] the percutaneously absorbable preparation described in [1] above, wherein the Cmax is 0.05 to 0.4 ng/ml·cm²;
[4] the percutaneously absorbable preparation described in any of [1] to [3] above, wherein AUC per unit surface area of the percutaneously absorbable preparation is 7.5 to 75 ng·hr/ml·cm²;
[5] the percutaneously absorbable preparation described in [4] above, wherein the AUC is 10 to 62.5 ng·hr/ml·cm²;
[6] the percutaneously absorbable preparation described in [4] above, wherein the AUC is 12.5 to 50 ng·hr/ml·cm²;
[7] the percutaneously absorbable preparation described in any of [1] to [3] above, wherein Tmax is 12 to 192 hr;
[8] the percutaneously absorbable preparation described in [7] above, wherein the Tmax is 24 to 180 hr;
[9] the percutaneously absorbable preparation described in [7] above, wherein the Tmax is 48 to 168 hr;
[10] the percutaneously absorbable preparation described in any of [1] to [3] above, wherein AUC per unit surface area of the percutaneously absorbable preparation is 7.5 to 75 ng·hr/ml·cm², and Tmax is 12 to 192 hr;
[11] the percutaneously absorbable preparation described in [10] above, wherein the Tmax is 24 to 180 hr;
[12] the percutaneously absorbable preparation described in [10] above, wherein the Tmax is 48 to 168 hr;
[13] the percutaneously absorbable preparation described in any of [1] to [3] above, wherein AUC per unit surface area of the percutaneously absorbable preparation is 10 to 62.5 ng·hr/ml·cm², and Tmax is 12 to 192 hr;
[14] the percutaneously absorbable preparation described in [13] above, wherein the Tmax is 24 to 180 hr;
[15] the percutaneously absorbable preparation described in [13] above, wherein the Tmax is 48 to 168 hr;
[16] the percutaneously absorbable preparation described in any of [1] to [3] above, wherein AUC per unit surface area of the percutaneously absorbable preparation is 12.5 to 50 ng·hr/ml·cm², and Tmax is 12 to 192 hr;
[17] the percutaneously absorbable preparation described in [16] above, wherein the Tmax is 24 to 180hr;
[18] the percutaneously absorbable preparation described in [16] above, wherein the Tmax is 48 to 168 hr;
[19] a percutaneously absorbable preparation comprising 2-[(1-benzylpiperidin-4-yl) methyl]-5,6-dimethoxyindan-1-one and/or a hydrochloride thereof,
   wherein the percutaneously absorbable preparation js able to administer to a patient such that AUC per unit surface area of the percutaneously absorbable preparation is 7.5 to 75 ng·hr/ml·cm² in a plasma concentration profile;
[20] the percutaneously absorbable preparation described in [19] above, wherein the AUC is 10 to 62.5 ng·hr/ml·cm²;
[21] the percutaneously absorbable preparation described in [19] above, wherein the AUC is 12.5 to 50 ng·hr/ml·cm²;
[22] the percutaneously absorbable preparation described in any of [19] to [21] above, wherein Tmax is 12 to 192 hr;
[23] the percutaneously absorbable preparation described in [22] above, wherein the Tmax is 24 to 180 hr;
[24] the percutaneously absorbable preparation described in [22] above, wherein the Tmax is 48 to 168 hr;
[25] a percutaneously absorbable preparation comprising 2-[(1-benzylpiperidin-4-yl) methyl]-5,6-dimethoxyindan-1-one and/or a hydrochloride thereof, capable of being administered to a patient such that Tmax of the plasma concentration profile is 12 to 192 hr;
[26] the percutaneously absorbable preparation described in [25] above, wherein the Tmax is 24 to 180 hr;
[27] the percutaneously absorbable preparation described in [25] above, wherein the Tmax is 48 to 168 hr;
[28] the percutaneously absorbable preparation described in [1] above, which is applied to 5 to 150 cm² of the skin of the patient; and
[29] the percutaneously absorbable preparation described in [1] above, which is applied to the patient one to seven times per week.

### Advantageous Effects of the Invention

According to the present invention, a percutaneously absorbable preparation comprising donepezil and/or donepezil hydrochloride is provided that demonstrates a favorable plasma drug concentration profile.

### BRIEF DESCRIPTION OF THE DRAWINGS

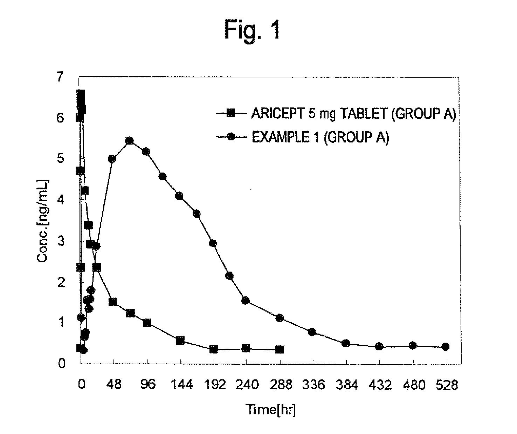

FIG. 1 shows changes in concentrations of donepezil in plasma following oral administration of an Aricept 5 mg tablet in a single-dose administration test on healthy adults carried out in Example 3 or adhesion of a percutaneously absorbable preparation of Example 1. The horizontal axis indicates elapsed time after administration, while the vertical axis indicates plasma drug concentrations. In the graph, ◆ indicate blood collection times and plasma concentrations at those times in the case of administration of an Aricept 5 mg tablet, while ● indicate blood collection times and plasma concentrations at those times following adhesion of the percutaneously absorbable preparation of Example 1.

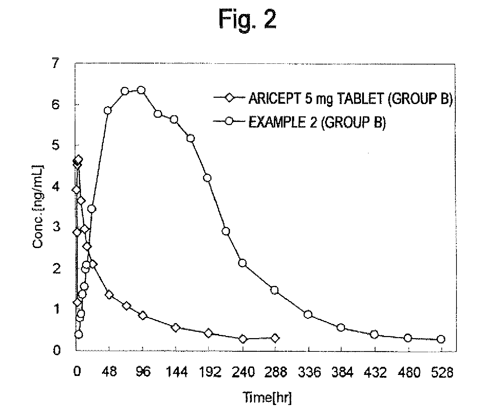

FIG. 2 shows changes in concentrations of donepezil in plasma following oral administration of an Aricept 5 mg tablet in a single-dose administration test on healthy adults carried out in Example 4 or adhesion of a percutaneously absorbable preparation of Example 2. The horizontal axis indicates elapsed time after administration, while the vertical axis indicates plasma drug concentrations. In the graph, 0 indicate blood collection times and plasma concentrations at those times in the case of administration of an Aricept 5 mg tablet, while 0 indicate blood collection times and plasma concentrations at those times following adhesion of the percutaneously absorbable preparation of Example 2.

### DETAILED DESCRIPTION

The following provides a more detailed explanation of the present invention.

The percutaneously absorbable preparation of the present invention is for percutaneous absorption of donepezil and/or donepezil hydrochloride (hereinafter, "donepezil and/or donepezil hydrochloride" may be abbreviated as "donepezil and the like"). The percutaneously absorbable preparation of the present invention enables an increase in the plasma concentration of donepezil and the like following adhesion of the percutaneously absorbable preparation to be gradual by demonstrating the profile described below for the plasma drug concentration of the donepezil and the like. In addition, the percutaneously absorbable preparation of the present invention is a percutaneously absorbable preparation that imparts sustainability to the effective plasma concentration of donepezil and the like as compared to oral administration of a tablet and the like, thereby eliminating the need for frequent changing.

In particular, the percutaneously absorbable preparation of the present invention can be expected to reduce the incidences of adverse side effects by controlling the Cmax per unit surface area of the percutaneously absorbable preparation to the range of the present invention, In addition, the plasma concentration of donepezil and the like in the steady state in the case of having administered a tablet can be maintained by the percutaneously absorbable preparation having a practical preparation size by controlling the Cmax and AUC per unit surface area of the percutaneously absorbable preparation to the ranges of the present invention.

The percutaneously absorbable preparation of the present invention is for allowing donepezil and the like percutaneously absorbable, and is a percutaneously absorbable preparation that enables donepezil and the like to be administered to a patient so that the Cmax per unit surface area of the percutaneously absorbable preparation for donepezil and the like is 0.025 to 0.5 ng/ml·cm² in the plasma concentration profile.

There are no particular limitations on the percutaneously absorbable preparation of the present invention, provided it is a preparation that enables donepezil and the like to be administered to a patient such that the Cmax per unit surface area of the percutaneously absorbable preparation is 0.025 to 0.5 ng/m·cm² in the plasma concentration profile for donepezil and the like. For example, the preparation may be a preparation that enables the Cmax to be achieved by a single administration, or a preparation that enables a Cmax similar to the above-mentioned Cmax to be achieved by multiple administrations.

The percutaneously absorbable preparation of the present invention may be a percutaneously absorbable preparation that enables donepezil and the like to be administered to a patient such that the Cmax per unit surface area of the percutaneously absorbable preparation in a single administration is 0.025 to 0.5 ng/ml·cm² in the plasma concentration profile for donepezil and the like, or may be a percutaneously absorbable preparation that enables donepezil and the like to be administered to a patient such that the Cmax per unit surface area of the percutaneously absorbable preparation required to demonstrate the plasma concentration profile of the present invention during multiple administrations is 0.025 to 0.5 ng/ml·cm².

In the present invention, the term "Cmax (ng/ml)" refers to the maximum plasma drug concentration. The term "maximum plasma drug concentration" refers to the maximum value of the concentration of a drug in the plasma following administration of that drug.

In the present invention, the term "Cmax per unit surface area of the percutaneously absorbable preparation" refers to the value obtained by dividing the Cmax in the case of administering the percutaneously absorbable preparation of the present invention by the surface area of the portion of the percutaneously absorbable preparation in contact with a patient's skin.

In the present invention, the term "Cmax per unit surface area of the percutaneously absorbable preparation in a single administration" refers to the value obtained by dividing the Cmax in the case of a single administration of the percutaneously absorbable preparation of the present invention by the surface area of the portion of the percutaneously absorbable preparation in contact with a patient's skin.

In the present invention, the term "Cmax per unit surface area of the percutaneously absorbable preparation during multiple administrations" refers to the value obtained by dividing the Cmax of the percutaneously absorbable preparation of the present invention during multiple administrations by the surface area of the portion of the percutaneously absorbable preparation in contact with a patient's skin.

In the percutaneously absorbable preparation of the present invention, the Cmax per unit surface area of the percutaneously absorbable preparation is preferably 0.25 to 0.45 ng/ml·cm² and more preferably 0.05 to 0.4 ng/ml·cm².

The percutaneously absorbable preparation of the present invention is a percutaneously absorbable preparation that enables donepezil and the like to be administered to a patient so that the AUC per unit surface area of the percutaneously absorbable preparation is 7.5 to 75 ng·hr/ml·cm² in the plasma concentration profile for donepezil and the like.

There are no particular limitations on the percutaneously absorbable preparation of the present invention, provided it is a preparation that enables donepezil and the like to be administered to a patient such that the AUC per unit surface area of the percutaneously absorbable preparation is 7.5 to 75 ng·hr/ml·cm² . For example, the preparation may be a preparation that enables the above-mentioned AUC to be achieved by a single administration, or a preparation that enables an AUC similar to the above-mentioned AUC to be achieved by multiple administrations.

The percutaneously absorbable preparation of the present invention may be a percutaneously absorbable preparation that enables donepezil and the like to be administered to a patient such that the AUC per unit surface area of the percutaneously absorbable preparation in a single administration is 7.5 to 75 ng·hr/ml·cm² in the plasma concentration profile for donepezil and the like, or may be a percutaneously absorbable preparation that enables donepezil and the like to be administered to a patient such that the AUC per unit surface area of the percutaneously absorbable preparation during multiple administrations which are required to demonstrate the plasma concentration profile of the present invention is 7.5 to 75 ng·hr/ml·cm².

In the present invention, the term "AUC (ng·hr/ml·cm²)" refers to the area under a drug concentration versus time curve. The term "area under a drug concentration versus time curve" refers to the area of a portion encompassed by a curve depicted on a graph representing elapsed time of plasma drug concentration (plasma drug concentration versus time curve) and the horizontal axis (time axis).

In the present invention, the term "AUC per unit surface area of the percutaneously absorbable preparation" refers to the value obtained by dividing the AUC in the case of administering the percutaneously absorbable preparation of the present invention by the surface area of the portion of the percutaneously absorbable preparation in contact with a patient's skin.

In the present invention, the term "AUC per unit surface area of the percutaneously absorbable preparation in a single administration" refers to the value obtained by dividing the AUC in the case of a single administration of the percutaneously absorbable preparation of the present invention by the surface area of the portion of the percutaneously absorbable preparation in contact with a patient's skin.

In the present invention, the term "AUC per unit surface area of the percutaneously absorbable preparation during multiple administrations" refers to the value obtained by dividing the AUC of the percutaneously absorbable preparation of the present invention during multiple administrations by the surface area of the portion of the percutaneously absorbable preparation in contact with a patient's skin.

In the present invention, the term "AUC" refers to either AUCinf or AUClast, and although there are no particular limitations thereon, AUCinf is preferable.

The term "AUCinf" refers to the value of AUC for a plasma drug concentration obtained by extrapolating time to infinity based on the elimination rate from the blood.

The term "AUClast" refers to the value of AUC up to the last measurement point on the plasma drug concentration versus time curve. In the present invention, the term "AUClast" is preferably AUC_{0→528}.

In the percutaneously absorbable preparation of the present invention, the AUC per unit surface area of the percutaneously absorbable preparation is preferably 10 to 62.5 ng·hr/ml·cm² and more preferably 12.5 to 50 ng·hr/ml·cm².

The percutaneously absorbable preparation of the present invention is a percutaneously absorbable preparation that enables donepezil and the like to be administered to a patient so that the Tmax of the plasma concentration profile for donepezil and the like is 12 to 192 hr.

There are no particular limitations on the percutaneously absorbable preparation of the present invention, provided it is a preparation that enables donepezil and the like to be administered to a patient such that the Tmax is 12 to 192 hr. For example, the preparation may be a preparation that enables the above-mentioned Tmax to be achieved by a single administration, or a preparation that enables a Tmax similar to the above-mentioned Tmax to be achieved by multiple administrations.

The percutaneously absorbable preparation of the present invention may be a percutaneously absorbable preparation that enables donepezil and the like to be administered to a patient such that the Tmax of the plasma concentration profile for donepezil and the like in a single administration is 12 to 192 hr, or may be a percutaneously absorbable preparation that enables donepezil and the like to be administered to a patient such that the Tmax during multiple administrations which is required to demonstrate the plasma concentration profile of the present invention is 12 to 192 hr.

In the present invention, the term "Tmax" refers to the maximum plasma concentration attainment time. The term "the maximum plasma concentration attainment time" refers to the time required for the concentration of a drug in the plasma to reach a maximum following administration of that drug.

In the present invention, Tmax is the value of Tmax in the case of having administered the percutaneously absorbable preparation of the present invention.

In the present invention, the term "Tmax in a single administration" refers to the value of Tmax in the case of having administered a single administration of the percutaneously absorbable preparation of the present invention.

In the present invention, the term "Tmax during multiple administrations" refers to the value of Tmax in the case of having administered multiple administrations of the percutaneously absorbable preparation of the present invention.

In the percutaneously absorbable preparation of the present invention, Tmax is preferably 24 to 180 hr and more preferably 48 to 168 hr.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², and the AUC per unit surface area of the percutaneously absorbable preparation is 7.5 to 75 ng·hr/ml·cm² in the plasma concentration profile for donepezil and the like.

The percutaneously absorbable preparation of the present invention is more preferably a percutaneously absorbable preparation that can be administered to a patient so that the Cmax per unit surface area of the percutaneously absorbable preparation is 0.025 to 0.45 ng/ml·cm², and the AUC per unit surface area of the percutaneously absorbable preparation is 7.5 to 75 ng·hr/ml·cm² in the plasma concentration profile for donepezil and the like.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², and the AUC per unit surface area of the percutaneously absorbable preparation is 7.5 to 75 ng·hr/ml·cm² in the plasma concentration profile for donepezil and the like.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², and the AUC per unit surface area of the percutaneously absorbable preparation is 10 to 62.5 ng·hr/ml·cm² in the plasma concentration profile for donepezil and the like.

The percutaneously absorbable preparation of the present invention is more preferably a percutaneously absorbable preparation that can be administered to a patient so that the Cmax per unit surface area of the percutaneously absorbable preparation is 0.025 to 0.45 ng/ml·cm², and the AUC per unit surface area of the percutaneously absorbable preparation is 10 to 62.5 ng·hr/ml·cm² in the plasma concentration profile for donepezil and the like.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², and the AUC per unit surface area of the percutaneously absorbable preparation is 10 to 62.5 ng·hr/ml-cm² in the plasma concentration profile for donepezil and the like.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², and the AUC per unit surface area of the percutaneously absorbable preparation is 12.5 to 50 ng·hr/ml·cm² in the plasma concentration profile for donepezil and the like.

The percutaneously absorbable preparation of the present invention is more preferably a percutaneously absorbable preparation that can be administered to a patient so that the Cmax per unit surface area of the percutaneously absorbable preparation is 0.025 to 0.45 ng/ml·cm², and the AUC per unit surface area of the percutaneously absorbable preparation is 12.5 to 50 ng·hr/ml·cm² in the plasma concentration profile for donepezil and the like.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², and the AUC per unit surface area of the percutaneously absorbable preparation is 12.5 to 50 ng·hr/ml·cm² in the plasma concentration profile for donepezil and the like.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², and the Tmax is 12 to 192 hr in the plasma concentration profile for donepezil and the like.

The percutaneously absorbable preparation of the present invention is more preferably a percutaneously absorbable preparation that can be administered to a patient so that the Cmax per unit surface area of the percutaneously absorbable preparation is 0.025 to 0.45 ng/ml·cm², and the Tmax is 12 to 192 hr in the plasma concentration profile for donepezil and the like.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², and the Tmax is 12 to 192 hr in the plasma concentration profile for donepezil and the like.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², and the Tmax is 24 to 150 hr in the plasma concentration profile for donepezil and the like.

The percutaneously absorbable preparation of the present invention is more preferably a percutaneously absorbable preparation that can be administered to a patient so that the Cmax per unit surface area of the percutaneously absorbable preparation is 0.025 to 0.45 ng/ml·cm², and the Tmax is 24 to 180 hr in the plasma concentration profile for donepezil and the like.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², and the Tmax is 24 to 180 hr in the plasma concentration profile for donepezil and the like.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², and the Tmax is 48 to 168 hr in the plasma concentration profile for donepezil and the like.

The percutaneously absorbable preparation of the present invention is more preferably a percutaneously absorbable preparation that can be administered to a patient so that the Cmax per unit surface area of the percutaneously absorbable preparation is 0.025 to 0.45 ng/ml·cm², and the Tmax is 48 to 168 hr in the plasma concentration profile for donepezil and the like.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², and the Tmax is 48 to 168 hr in the plasma concentration profile for donepezil and the like.

The percutaneously absorbable preparation of the present invention is preferably a percutaneously absorbable preparation that can be administered to a patient so that the AUC per unit surface area of the percutaneously absorbable preparation is 7.5 to 75 ng·hr/ml·cm² , and the Tmax is 12 to 192 hr in the plasma concentration profile for donepezil and the like.

The percutaneously absorbable preparation of the present invention is more preferably a percutaneously absorbable preparation that can be administered to a patient so that the AUK per unit surface area of the percutaneously absorbable preparation is 10 to 62.5 ng·hr/ml·cm², and the Tmax is 12 to 192 hr in the plasma concentration profile for donepezil and the like.

The percutaneously absorbable preparation of the present invention is even more preferably a percutaneously absorbable preparation that can be administered to a patient so that the AUC per unit surface area of the percutaneously absorbable preparation is 12.5 to 50 ng·hr/ml·cm², and the Tmax is 12 to 192 hr in the plasma concentration profile for donepezil and the like.

The percutaneously absorbable preparation of the present invention is preferably a percutaneously absorbable preparation that can be administered to a patient so that the AUC per unit surface area of the percutaneously absorbable preparation is 7.5 to 75 ng·hr/ml·cm², and the Tmax is 24 to 180 hr in the plasma concentration profile for donepezil and the like.

The percutaneously absorbable preparation of the present invention is more preferably a percutaneously absorbable preparation that can be administered to a patient so that the AUC per unit surface area of the percutaneously absorbable preparation is 10 to 62.5 ng·hr/ml·cm², and the Tmax is 24 to 180 hr in the plasma concentration profile for donepezil and the like.

The percutaneously absorbable preparation of the present invention is even more preferably a percutaneously absorbable preparation that can be administered to a patient so that the AUC per unit surface area of the percutaneously absorbable preparation is 12.5 to 50 ng·hr/ml·cm², and the Tmax is 24 to 180 hr in the plasma concentration profile for donepezil and the like.

The percutaneously absorbable preparation of the present invention is preferably a percutaneously absorbable preparation that can be administered to a patient so that the AUC per unit surface area of the percutaneously absorbable preparation is 7.5 to 75 ng·hr/ml·cm², and the Tmax is 48 to 168 hr in the plasma concentration profile for donepezil and the like.

The percutaneously absorbable preparation of the present invention is more preferably a percutaneously absorbable preparation that can be administered to a patient so that the AUC per unit surface area of the percutaneously absorbable preparation is 10 to 62.5 ng·hr/ml·cm², and the Tmax is 48 to 168 hr in the plasma concentration profile for donepezil and the like.

The percutaneously absorbable preparation of the present invention is even more preferably a percutaneously absorbable preparation that can be administered to a patient so that the AUC per unit surface area of the percutaneously absorbable preparation is 12.5 to 50 ng·hr/ml·cm², and the Tmax is 48 to 168 hr in the plasma concentration profile for donepezil and the like.

The percutaneously absorbable preparation of the present invention is preferably a percutaneously absorbable preparation that can be administered to a patient so that the Cmax per unit surface area of the percutaneously absorbable preparation is 0.025 to 0.5 ng/ml·cm², the AUC per unit surface area of the percutaneously absorbable preparation is 7.5 to 75 ng·hr/ml·cm², and the Tmax is 12 to 192 hr in the plasma concentration profile for donepezil and the like.

In the percutaneously absorbable preparation of the present invention, the Cmax per unit surface area of the above-mentioned percutaneously absorbable preparation is more preferably 0.025 to 0.45 ng/ml·cm² and even more preferably 0.05 to 0.4 ng/ml·cm² in the plasma concentration profile for donepezil and the like defined by Cmax, AUC and Tmax.

In the percutaneously absorbable preparation of the present invention, the AUC per unit surface area of the above-mentioned percutaneously absorbable preparation is preferably 10 to 62.5 ng·hr/ml·cm² and more preferably 12.5 to 50 ng·hr/ml·cm² in the plasma concentration profile for donepezil and the like defined by Cmax, AUC and Tmax.

In the percutaneously absorbable preparation of the present invention, the Tmax of the above-mentioned percutaneously absorbable preparation is preferably 24 to 180 hr and more preferably 48 to 168 hr in the plasma concentration profile for donepezil and the like defined by Cmax, AUC and Tmax.

The percutaneously absorbable preparation of the present invention is even more preferably a percutaneously absorbable preparation that can be administered to a patient so that the Cmax per unit surface area of the percutaneously absorbable preparation is 0.05 to 0.4 ng/ml·cm², the AUC per unit surface area of the percutaneously absorbable preparation is 12.5 to 50 ng·hr/ml·cm², and the Tmax is 48 to 188 hr in the plasma concentration profile for donepezil and the like.

In the present invention, the term "Tlag" refers to the time until a drug is absorbed into the body and appears in the plasma following administration of that drug.

The percutaneously absorbable preparation of the present invention can be used as an anti-Alzheimer's dementia drug. In addition, other possible applications include use against cerebrovascular dementia, prevention of migraine headaches and the like.

In the present invention, a patient refers to a mammal, and preferably a human. Patients include men and women as well as infants, children and adults. In particular, patients include persons suffering from Alzheimer's dementia, cerebrovascular dementia and/or migraine headaches.

There are no particular limitations on the percutaneously absorbable preparation in the present invention, provided it enables donepezil or donepezil hydrochloride to be absorbed through the skin.

Examples of application sites of the percutaneously absorbable preparation of the present invention include adhered sites where ordinary patches are used, such as the back, chest, upper arm and thigh.

The percutaneously absorbable preparation of the present invention is preferably a percutaneously absorbable preparation having a pressure-sensitive adhesive layer in order to demonstrate the preferable plasma drug concentration for donepezil and the like in the present invention. Specifically, the percutaneously absorbable preparation of the present invention is preferably a percutaneously absorbable preparation having a pressure-sensitive adhesive layer comprising donepezil and/or donepezil hydrochloride that enables donepezil and the like to be administered to a patient so as to demonstrate the preferable plasma concentration profile for donepezil and the like in the present invention.

The percutaneously absorbable preparation of the present invention is preferably a percutaneously absorbable preparation in which the pressure-sensitive adhesive layer is crosslinked. The percutaneously absorbable preparation of the present invention is preferably a percutaneously absorbable preparation in which the pressure-sensitive adhesive layer is crosslinked using a crosslinking agent. The percutaneously absorbable preparation of the present invention is preferably a percutaneously absorbable preparation in which the pressure-sensitive adhesive layer comprises an acrylic pressure-sensitive adhesive. The percutaneously absorbable preparation of the present invention is preferably a percutaneously absorbable preparation in which the pressure-sensitive adhesive layer further comprises a liquid plasticizer. The percutaneously absorbable preparation of the present invention is preferably a percutaneously absorbable preparation in which the pressure-sensitive adhesive layer further comprises a metal chloride. The percutaneously absorbable preparation of the present invention is preferably a percutaneously absorbable preparation in which the thickness of the pressure-sensitive adhesive layer is 20 to 300 µm. The percutaneously absorbable preparation of the present invention is preferably a percutaneously absorbable preparation comprising as an active ingredient thereof 2 to 250 mg of donepezil and the like.

In addition, the percutaneously absorbable preparation of the present invention is preferably a percutaneously absorbable preparation that is applied to 5 to 150 cm² of the skin of a patient in order to demonstrate the preferable plasma concentration profile for donepezil and the like of the present invention. The percutaneously absorbable preparation of the present invention is preferably a percutaneously absorbable preparation that is applied 1 to 7 times per week to a patient in order to demonstrate the preferable plasma concentration profile for donepezil and the like in the present invention.

In the percutaneously absorbable preparation of the present invention, the content of donepezil and/or donepezil hydrochloride in the pressure-sensitive adhesive layer is normally 1 to 30 wt% and preferably 3 to 20 wt% in order to demonstrate the preferable plasma concentration profile for donepezil and the like in the present invention. In the case the content is less than 1 wt%, an amount effective for treatment may not be released, while in the case the content exceeds 30 wt%, there is uneconomical without therapeutic benefits .

In the percutaneously absorbable preparation of the present invention, there are no particular limitations on the pressure-sensitive adhesive that forms the pressure-sensitive adhesive layer, provided it has the ability to crosslink, examples of which include acrylic pressure-sensitive adhesives; rubber pressure-sensitive adhesives such as silicone rubber, polyisoprene rubber, polyisobutylene rubber, styrene-butadiene rubber, styrene-isoprene-styrene block copolymer rubber or styrene-butadiene-styrene block copolymer rubber; silicone pressure-sensitive adhesives; and vinyl polymer pressure-sensitive adhesives such as polyvinyl alcohol, polyvinyl alkyl ether or polyvinyl acetate.

In the percutaneously absorbable preparation of the present invention, the pressure-sensitive adhesive layer is preferably crosslinked, and for example, a known chemical crosslinking treatment or physical crosslinking treatment can be carried out (for example, physical crosslinking by irradiating with gamma rays or other electron beam or irradiating with ultraviolet light, or chemical crosslinking by adding a crosslinking agent). Specifically, the percutaneously absorbable preparation of the present invention is preferably a percutaneously absorbable preparation having a crosslinked pressure-sensitive adhesive layer and which can be administered to a patient so as to demonstrate the preferable plasma concentration profile for donepezil and the like in the present invention. In addition, the percutaneously absorbable preparation of the present invention is more preferably a percutaneously absorbable preparation having a pressure-sensitive adhesive layer crosslinked by using a crosslinking agent and which can be administered to a patient so as to demonstrate the preferable plasma concentration profile for donepezil and the like in the present invention.

In the present invention, a functional group capable of being involved in a crosslinking reaction such as hydroxyl groups, carboxyl groups or vinyl groups are preferably introduced into the pressure-sensitive adhesive, and introduction of functional groups capable of being involved in a crosslinking reaction into the pressure-sensitive adhesive is carried out by a known method. For example, during synthesis of a polymer to serve as the pressure-sensitive adhesive, introduction of functional groups can be carried out by adding a monomer having a hydroxyl group such as hydroxymethyl (meth)acrylate or monomer having a carboxyl group such as acrylic acid or maleic acid followed by copolymerization thereof. Furthermore, crosslinking of the pressure-sensitive adhesive may be carried out by adding a monomer and the like having two or more vinyl groups such as divinyl benzene or ethylene glycol dimethacrylate and copolymerizing during synthesis of the polymer serving as the pressure-sensitive adhesive to form intermolecular or intramolecular crosslinks during the polymerization reaction.

Among the examples of pressure-sensitive adhesives used in the present invention as described above, an acrylic pressure-sensitive is preferable from the viewpoint of facilitating crosslinking treatment and favorable adhesion to the skin as a percutaneously absorbable preparation, and to demonstrate the preferable plasma concentration profile for donepezil and the like of the present invention. An acrylic pressure-sensitive adhesive is particularly preferable for enabling Cmax to demonstrate the preferable plasma concentration profile in the present invention. Although Cmax is affected by various components of the pressure-sensitive adhesive layer, it is thought to be primarily dependent on the concentration of donepezil and the like in the pressure-sensitive adhesive layer. Since donepezil and the like dissolves easily in acrylic pressure-sensitive adhesives, the pressure-sensitive adhesive is preferably an acrylic pressure-sensitive adhesive in order to demonstrate the Cmax as previously described.

Specifically, the percutaneously absorbable preparation of the present invention is preferably a percutaneously absorbable preparation having a pressure-sensitive adhesive layer comprising an acrylic pressure-sensitive adhesive and which can be administered to a patient so as to demonstrate the preferable plasma concentration profile for donepezil and the like in the present invention.

The acrylic pressure-sensitive adhesive of the present invention is normally an acrylic pressure-sensitive adhesive comprising a (meth)acrylic acid alkyl ester, and is preferably an acrylic pressure-sensitive adhesive having a (meth)acrylic acid alkyl ester as a principal component (principal constituent unit) thereof. A copolymer of a (meth)acrylic acid alkyl ester (first monomer component) as the principal component with a vinyl monomer having functional groups capable of contributing to a crosslinking reaction (second monomer component), or a copolymer of these copolymerized with yet another monomer (third monomer component), is particularly preferable from the viewpoint of ease of crosslinking, pressure-sensitive adhesiveness such as adhesiveness with human skin, ability to manipulate drug dissolution and the like.

Preferable examples of the (meth)acrylic acid alkyl ester (first monomer component) include (meth)acrylic acid alkyl esters wherein the alkyl group is a linear, branched or cyclic alkyl group having 1 to 18 carbon atoms (such as methyl, ethyl, propyl, butyl, pentyl, hexyl, cyclohexyl, heptyl, octyl, 2-ethylhexyl, nonyl, decyl, undecyl, dodecyl or tridecyl), and (meth)acrylic acid alkyl esters wherein the alkyl group is a linear, branched or cyclic alkyl group having 4 to 18 carbon atoms (such as butyl, pentyl, hexyl, cyclohexyl, heptyl, octyl, 2-ethylhexyl, nonyl, decyl, undecyl, dodecyl or tridecyl). Moreover, since the use of a monomer component that lowers the glass transition temperature of the polymer is more preferable for imparting pressure-sensitive adhesiveness at normal temperatures, a (meth)acrylic acid alkyl ester wherein the alkyl group is a linear, branched or cyclic alkyl group having 4 to 8 carbon atoms (such as butyl, pentyl, hexyl, cyclohexyl, heptyl, octyl or 2-ethylhexyl, preferably butyl, 2-ethylhexyl or cyclohexyl and particularly preferably 2-ethylhexyl) is more preferable. More specifically, butyl acrylate, 2-ethylhexyl acrylate, 2-ethylhexyl methacrylate, cyclohexyl acrylate, cyclohexyl methacrylate or the like is preferable, and among these, 2-ethylhexyl acrylate is most preferable. One of these (meth)acrylic acid alkyl esters (first monomer component) may be used, or two or more may be used in combination.

In the vinyl monomer (second monomer component) having functional groups capable of being involved in the crosslinking reaction, examples of functional groups capable of being involved in the crosslinking reaction include hydroxyl groups, carboxyl groups and vinyl groups, and hydroxyl groups and carboxyl groups are preferable. Specific examples of this monomer (second monomer component) include hydroxyethyl (meth)acrylate esters, hydroxypropyl (meth)acrylate esters, (meth)acrylic acid, itaconic acid, maleic acid, maleic anhydride, mesaconic acid, citraconic acid and glutaconic acid. Among these, acrylic acid, methacrylic acid and a hydroxyethyl acrylate ester (particularly 2-hydroxyethyl acrylate) are preferable from the viewpoint of availability, and acrylic acid is most preferable. One of these monomers (second monomer component) may be used, or two or more may be used in combination.

The above-mentioned other monomer (third monomer component) is used primarily to adjust the cohesiveness of the pressure-sensitive adhesive layer and to adjust the solubility or release properties of the donepezil and the like. Examples of this monomer (third monomer component) include vinyl acetate, vinyl propionate and other vinyl esters; methyl vinyl ether, ethyl vinyl ether and other vinyl ethers; N-vinyl-2-pyrrolidone, N-vinyl caprolactam and other vinyl amides; methoxyethyl (meth)acrylate ester, ethoxyethyl (meth)acrylate ester, tetrahydrofurfuryl (meth)acrylate ester and other alkoxy (meth)acrylate esters; hydroxypropyl (meth)acrylate, α-hydroxymethyl acrylate and other hydroxyl group-containing monomers (which do not provide crosslinking sites because they are used as the third monomer component); (meth)acrylamide, dimethyl (meth)acrylamide, N-butyl (meth)acrylamide, N-methylol (meth)acrylamide and other (meth)acrylic acid derivatives having amide groups; aminoethyl (meth)acrylate ester, dimethylaminoethyl (meth)acrylate ester, t-butylaminoethyl (meth)acrylate ester and other aminoalkyl (meth)acrylate esters; methoxyethylene glycol (meth)acrylate ester, methoxydiethylene glycol (meth)acrylate ester, methoxypolyethylene glycol (meth)acrylate ester, methoxypolypropylene glycol (meth)acrylate ester and other alkoxyalkylene glycol (meth)acrylate esters; (meth)acrylonitrile; styrene sulfonic acid, allyl sulfonic acid, sulfopropyl (meth)acrylate, (meth)acryloyl oxynaphthalene sulfonic acid, acrylamide methyl sulfonic acid and other monomers having sulfonic acid; and vinyl piperidone, vinyl pyrimidine, vinyl piperazine, vinyl pyrrole, vinyl imidazole, vinyl oxazole, vinyl morpholine and other vinyl group-containing monomers. Among these, a vinyl ester or vinyl amide is preferable, and vinyl acetate is preferable as a vinyl ester, while N-vinyl-2-pyrrolidone is preferable as a vinyl amide. One of these monomers (third monomer component) may be used or two or more may be used in combination.

When the acrylic pressure-sensitive adhesive is a copolymer of a (meth)acrylic acid alkyl ester (first monomer component) and a vinyl monomer having functional groups capable of being involved in a crosslinking reaction (second monomer component), the (meth)acrylic acid alkyl ester and the vinyl monomer having functional groups capable of being involved in a crosslinking reaction are blended and copolymerized preferably at a weight ratio of 99 to 85 parts of (meth)acrylic acid alkyl ester per 1 to 15 parts of vinyl monomer having functional groups capable of being involved in a crosslinking reaction, and more preferably at a weight ratio of 99 to 90 parts per 1 to 10 parts.

In addition, when the acrylic pressure-sensitive adhesive is a copolymer of a (meth)acrylic acid alkyl ester (first monomer component), a vinyl monomer having functional groups capable of being involved in a crosslinking reaction (second monomer component) and another monomer (third monomer component), the (meth)acrylic acid alkyl ester, vinyl monomer having functional groups capable of being involved in a crosslinking reaction and other monomer are blended and copolymerized preferably at a weight ratio of 40 to 94 parts of (meth)acrylic acid alkyl ester per 1 to 15 parts of vinyl monomer having functional groups capable of being involved in a crosslinking reaction and 5 to 50 parts of other monomer, and more preferably at a weight ratio of 50 to 89 parts per 1 to 10 parts and 10 to 40 parts, respectively.

Although there are no particular limitations on the polymerization reaction, provided it is carried out with a known method, as an example thereof, a polymerization initiator (such as benzoyl peroxide, azobisisobutyronitrile or the like) is added to the above-mentioned monomers followed by reacting for 5 to 48 hours at 50 to 70°C in a solvent (such as ethyl acetate).

Particularly preferable examples of acrylic pressure-sensitive adhesives in the present invention include 2-ethylhexyl acrylate ester/acrylic acid/N-vinyl-2-pyrrolidone copolymer, 2-ethylhexyl acrylate ester/2-hydroxyethyl acrylate ester/vinyl acetate copolymer and 2-ethylhexyl acrylate ester/acrylic acid copolymer, while a more preferable example is 2-ethylhexyl acrylate ester/acrylic acid/N-vinyl-2-pyrrolidone copolymer.

In addition, although varying according to the copolymer composition, the glass transition temperature of the acrylic pressure-sensitive adhesive in the present invention is normally preferably -100 to -10°C and more preferably -90 to -20°C from the viewpoint of adhesiveness of the percutaneously absorbable preparation.

In the percutaneously absorbable preparation of the present invention, a liquid plasticizer can be comprised in the pressure-sensitive adhesive layer from the viewpoint of imparting softness to the pressure-sensitive adhesive layer and reducing pain and alleviating skin irritation caused by skin adhesiveness when the percutaneously absorbable preparation is peeled off the skin, and in order to demonstrate the preferable plasma concentration profile for donepezil and the like in the present invention. Specifically, the percutaneously absorbable preparation of the present invention is preferably a percutaneously absorbable preparation having a pressure-sensitive adhesive layer comprising a liquid plasticizer, and which can be administered to a patient so as to demonstrate the preferable plasma concentration profile for donepezil and the like in the present invention. Although the liquid plasticizer can be used without any particular limitations provided it is a liquid at room temperature, exhibits a plasticizing action and is compatible with the pressure-sensitive adhesive polymers composing the pressure-sensitive adhesive, that which improves the percutaneous absorbability and storage stability of donepezil and the like is preferable. In addition, the liquid plasticizer can also be incorporated for the purpose of further enhancing the solubility of donepezil and the like in the pressure-sensitive adhesive and the like. Examples of such liquid plasticizers include fatty acid alkyl esters (such as esters of lower monovalent alcohols having 1 to 4 carbon atoms and saturated or unsaturated fatty acids having 12 to 16 carbon atoms); saturated or unsaturated fatty acids having 8 to 10 carbon atoms (such as caprylic acid (octanoic acid, C8), pelargonic acid (nonanoic acid, C9), capric acid (decanoic acid, C10) or lauric acid (C12)); ethylene glycol, diethylene glycol, triethylene glycol, polyethylene glycol, propylene glycol, polypropylene glycol and other glycols; olive oil, castor oil, squalene, lanoline and other oils and fats; ethyl acetate, ethyl alcohol, dimethyl decyl sulfoxide, decyl methyl sulfoxide, dimethyl sulfoxide, dimethyl formamide, dimethyl acetamide, dimethyl lauryl amide, dodecyl pyrrolidone, isosorbitol, oleyl alcohol and other organic solvents; liquid surfactants; diisopropyl adipate, phthalic acid esters, diethyl sebacate and other plasticizers; and liquid paraffin and other hydrocarbons. In addition, other examples include ethoxylated stearyl alcohol, glycerin esters (those liquid at room temperature), isotridecyl myristate, N-methylpyrrolidone, ethyl oleate, oleic acid, diisopropyl adipate, octyl palmitate, 1,3-propanediol and glycerin. Among these, a fatty acid alkyl ester, saturated fatty acid, hydrocarbon or organic solvent is preferable from the viewpoint of stability of the preparation and the like, and in particular, a fatty acid alkyl ester of a lower monovalent alcohol having 1 to 4 carbon atoms and a saturated or unsaturated fatty acid having 12 to 16 carbon atoms is more preferable from the view point of percutaneous absorbability. One of these liquid plasticizers may be used alone or two or more may be used in combination.

In addition, in the case of using an acrylic pressure-sensitive adhesive for the pressure-sensitive adhesive, the liquid plasticizer is preferably a fatty acid alkyl ester from the viewpoint of compatibility and the like with the acrylic pressure-sensitive adhesive, and is more preferably an ester of a lower monovalent alcohol having 1 to 4 carbon atoms and a saturated or unsaturated fatty acid having 12 to 16 carbon atoms. The saturated or unsaturated fatty acid having 12 to 16 carbon atoms is preferably a saturated fatty acid, while the lower monovalent alcohol having 1 to 4 carbon atoms may be either linear or branched. Preferable examples of fatty acids having 12 to 16 carbon atoms include lauric acid (C12), myristic acid (C14) and palmitic acid (C16), while preferable examples of lower monovalent alcohols having 1 to 4 carbon atoms include isopropyl alcohol, ethyl alcohol, methyl alcohol and propyl alcohol. Specific examples of particularly preferable fatty acid alkyl esters include isopropyl myristate, ethyl laurate and isopropyl palmitate. Specifically, the percutaneously absorbable preparation of the present invention is preferably a percutaneously absorbable preparation having a pressure-sensitive adhesive layer comprising an acrylic pressure-sensitive adhesive crosslinked with a crosslinking agent and a liquid plasticizer in the form of a fatty acid alkyl ester such as an ester of a lower monovalent alcohol having 1 to 4 carbon atoms and a saturated or unsaturated fatty acid having 12 to 16 carbon atoms, such as isopropyl myristate, and which can be administered to a patient so as to demonstrate the preferable plasma concentration profile for donepezil and the like in the present invention.

Furthermore, in the case of using a fatty acid alkyl ester, a fatty acid having 8 to 14 carbon atoms and/or glycerin may be used in combination with the fatty acid alkyl ester from the viewpoint of improving the percutaneous absorbability of the donepezil and the like.

The amount of the liquid plasticizer incorporated in the present invention is preferably 10 to 160 parts by weight and more preferably 40 to 150 parts by weight based on 100 parts by weight of the pressure-sensitive adhesive. If the incorporated amount is less than 10 parts by weight, favorable softness or the effect of reducing skin irritation may not be adequately obtained due to inadequate plasticization of the pressure-sensitive adhesive layer, while if the incorporated amount exceeds 160 parts by weight, the liquid plasticizer may not be retained in the pressure-sensitive adhesive even by the cohesive force of the pressure-sensitive adhesive, thereby resulting in the adhesive force being weakened due to blooming on the surface of the pressure-sensitive adhesive layer, and increasing the possibility of the preparation detaching from the skin surface during use.

In the percutaneously absorbable preparation of the present invention, although crosslinking treatment is carried out by a known chemical crosslinking treatment (such as crosslinking by adding a crosslinking agent) or physical crosslinking treatment (such as crosslinking by irradiating with gamma rays or other electron or irradiating or irradiating with ultraviolet light) as was previously described, this crosslinking treatment can be carried out by a technique commonly used in this field. Furthermore, crosslinking treatment by addition of a crosslinking agent is preferable from the viewpoint of reducing effects on the drug. In the present invention, the pressure-sensitive adhesive layer is preferably crosslinked in terms of retaining a required amount of liquid plasticizer in the pressure-sensitive adhesive layer without decreasing pressure-sensitive adhesiveness, and since adhesive properties of the pressure-sensitive adhesive layer are improved by crosslinking the pressure-sensitive adhesive layer, the pressure-sensitive adhesive layer is preferably crosslinked in consideration of the possibility of improving adhesiveness to the skin and affecting the plasma concentration profile for donepezil and the like.

In the case of carrying out a chemical crosslinking process using a crosslinking agent, there are no particular limitations on the crosslinking agent, provided crosslink formation by such a crosslinking agent is not inhibited in the presence of donepesil and the like, and examples thereof include peroxides (such as benzoyl peroxide (BPO) and the like), metal oxides (such as magnesium metasilicate aluminate and the like), polyfunctional isocyanate compounds, organic metal compounds (such as zirconium alaninate, zinc alaninate , zinc acetate, glycine ammonium zinc or titanium compounds), metal alcoholates (such as tetraethyl titanate, tetraisopropyl titanate, aluminum isopropylate or aluminum sec-butyrate) and metal chelate compounds (such as dipropoxy bis(acetylacetonate) titanium, tetraoctylene glycol titanium, aluminum isopropylate, ethylacetoacetate aluminum diisopropylate, aluminum tris(ethylacetoacetate) or aluminum tris(acetylacetonate)). Among these, a peroxide, metal oxide, organic metal compound, metal alcoholate or metal chelate compound is preferable, and a metal alcoholate or metal chelate compound is more preferable from the viewpoint of efficiently forming crosslinks in the presence of donepezil and/or donepezil hydrochloride, while a metal chelate compound is most preferable from the viewpoint of easily obtaining crosslinked structures with a suitable crosslinking density. In addition, among the metal chelate compounds, ethylacetoacetate aluminum diisopropylate is particularly preferable. One of these crosslinking agents may be used or two or more may be used in combination.

Although varying according to the type of crosslinking agent and pressure-sensitive adhesive, the incorporated amount of the crosslinking agent is generally 0.1 to 0.6 parts by weight and preferably 0.15 to 0.5 parts by weight based on 100 parts by weight of the pressure-sensitive adhesive. If the incorporated amount is less than 0.1 parts by weight, the crosslinking sites are too few to impart adequate cohesiveness to the pressure-sensitive adhesive layer, resulting in the risk of adhesive residue and strong skin irritation due to cohesive failure during peeling, while if the incorporated amount exceeds 0.6 parts by weight, although cohesiveness is large, there are cases in which adequate adhesiveness may be unable to be obtained. In addition, there is also the risk of skin irritation caused by residual unreacted crosslinking agent.

Chemical crosslinking treatment can be carried out by adding the crosslinking agent followed by heating at or above the crosslinking reaction temperature, and although the heating temperature at this time is suitably selected according to the type of crosslinking agent, it is preferably 60 to 90°C and more preferably 60 to 80°C. The heating time is preferably 12 to 96 hours and more preferably 24 to 72 hours.

In the percutaneously absorbable preparation of the present invention, it is preferable that a metal chloride is comprised together with donepezil and/or donepezil hydrochloride in the crosslinked pressure-sensitive adhesive layer. Comprising a metal chloride in the pressure-sensitive adhesive layer reduces the decrease in cohesiveness of the pressure-sensitive adhesive layer and the percutaneously absorbable preparation is attached to human skin, and makes it less likely for cohesive failure to occur when the pressure-sensitive adhesive layer is peeled off. Specifically, in the case of having a pressure-sensitive adhesive layer further comprising a metal chloride, the percutaneously absorbable preparation of the present invention is able to inhibit separation, removal and positional shifting of the percutaneously absorbable preparation from the skin caused by decreases in cohesiveness of the pressure-sensitive adhesive layer. Thus, the percutaneously absorbable preparation of the present invention is preferably a percutaneously absorbable preparation having a pressure-sensitive adhesive layer further comprising a metal chloride and which can be administered to a patient so as to demonstrate the preferable plasma concentration profile for donepezil and the like in the present invention.

There are no particular limitations on the metal chloride, and examples thereof include a chloride of an alkaline metal such as sodium or potassium; a chloride of an alkaline earth metal such as calcium or magnesium; aluminum chloride, stannous chloride and ferric chloride. From the viewpoint of superior stability and ability to inhibit decreases in cohesiveness of the pressure-sensitive adhesive layer, sodium chloride, calcium chloride, aluminum chloride, stannous chloride or ferric chloride is preferable, sodium chloride or calcium chloride is more preferable, and sodium chloride is particularly preferable. Any of these may be used alone or two or more may be used in combination.

The particle diameter of the metal chloride is generally broadly divided into a primary particle diameter and secondary particle diameter. In the case of observing the metal chloride under a light microscope or electron microscope, the primary particles are observed as small particles, while the secondary particles are observed in the form large particles formed by aggregation of a plurality of primary particles. In the present description, the respective maximum particle diameters thereof are referred to as primary particle diameter and secondary particle diameter. Although there are no particular limitations on these particle diameters of the metal chloride, a smaller particle diameter is preferable from the viewpoints of ease of handling during production as well as a favorable sense of adhesion and appearance attributable to smoothness of the adhered surface of the pressure-sensitive adhesive layer. Thus, the secondary particle diameter of the metal chloride is preferably 300 µm or less, more preferably 250 µm or less and most preferably 200 µm or less. Furthermore, although there are no particular limitations on the lower limit of secondary particle diameter, it is preferably 1 µm or more. If the secondary particle diameter is less than 1 µm, since the primary particle diameter of such a metal chloride is even smaller, there is a risk of a decrease in handling ease during production, such as the dispersal of particles.

Furthermore, although the secondary particles of the metal chloride are present in the pressure-sensitive adhesive layer in various forms such as flat or amorphous, since the particles were unexpectedly found to be easily comprised in the pressure-sensitive adhesive layer, even in cases in which the secondary particle size of the metal chloride is larger than the thickness of the pressure-sensitive adhesive layer, a preparation can be obtained that is resistant to decreases in smoothness of the adhered surface of the pressure-sensitive adhesive layer and is free of problems in terms of appearance provided the secondary particle diameter is a preferable particle diameter as previously described. As will be described later, in the case the support is a support having a comparatively high degree of flexibility in which paper, woven fabric or non-woven fabric and the like is comprised on the side of the pressure-sensitive adhesive layer, a portion of the secondary particles of the metal chloride can be present embedded in the support, and even in cases in which the secondary particle diameter of the metal chloride is larger than the thickness of the pressure-sensitive adhesive layer, a highly smooth adhered surface can be obtained extremely easily. When the support is comparatively lacking in flexibility, the secondary particle diameter of the metal chloride is preferably equal to or less than the thickness of the pressure-sensitive adhesive layer from the viewpoint of efficiently obtaining an adhered surface of adequately high smoothness, with the secondary particle diameter preferably being 1/2 or less, and particularly preferably being 1/3 or less, the thickness of the pressure-sensitive adhesive layer.

In the case the metal chloride is a salt formed by neutralizing donepezil hydrochloride with an inorganic base comprising a metal during the course of forming the pressure-sensitive adhesive layer described later, the preferable secondary particle diameter described above can be easily reproduced.

The particle diameters (primary particle diameter, secondary particle diameter) of the metal chloride in the percutaneously absorbable preparation of the present invention are values measured according to the measurement method described below.

The pressure-sensitive adhesive side of the pressure-sensitive adhesive layer of the percutaneously absorbable preparation (sample) is observed visually (furthermore, in the case a release sheet is present in the preparation, the release sheet is peeled off and the pressure-sensitive adhesive surface of the exposed pressure-sensitive adhesive layer is observed visually). Next, the sample is placed on the stage of a light microscope with the pressure-sensitive adhesive surface of the pressure-sensitive adhesive layer facing upward, and at least 20 particles are photographed under the light microscope together with a scale in order starting with those parties thought to be the largest based on visual observations. At this time, in the case the particles to be measured are primary particles, then at least 20 particles consisting of primary particles only are photographed, while in the case the particles to be measured are secondary particles, then at least 20 particles consisting of secondary particles only are photographed. Among the primary particles or secondary particles photographed, the partile diameter of a particle determined to be the particle having the largest particle diameter based on measuring with the scale is used as the primary particle diameter of secondary particle diameter, respectively. Furthermore, the "particle diameter" referred to here refers to the diameter of a circle externally tangent to a projected image of a particle (diameter equivalent to externally tangent circle). Furthermore, the "equivalent circumscribed circle diameter" is known to be defined as particle diameter for the sake of convenience during measurement of particle diameter by microscopic observation, and is described in, for example, "Powders - Theory and Application" (Revised 2nd edition, p. 55, published May 12, 1979, Maruzen Co., Ltd.).

The incorporated amount of the metal chloride is preferably 0.1 to 20 parts by weight, more preferably 1 to 15 parts by weight and most preferably 3 to 10 parts by weight based on 100 parts by weight of the pressure-sensitive adhesive. If the incorporated amount is less than 0.1 parts by weight, the effect of inhibiting decreases in cohesiveness of the pressure-sensitive adhesive layer may be inadequate, while conversely if the incorporated amount exceeds 20 parts by weight, although the inhibitory effect is demonstrated, the appearance of the preparation may be impaired due to the metal chloride not being uniformly dispersed in the pressure-sensitive adhesive (pressure-sensitive adhesive polymer).

However, since donepezil hydrochloride is more stable than donepezil and since donepezil has crystal polymorphism making it difficult to handle, donepezil hydrochloride is more advantageous than donepezil from the viewpoint of handling. On the other hand, the free form of donepezil has higher percutaneous absorbability than donepezil hydrochloride. In order to utilize both of these advantages, the metal chloride in the present invention may be a salt formed by neutralizing donepezil hydrochloride with an inorganic base comprising a metal during formation of the pressure-sensitive adhesive layer. This salt is equivalent to a metal chloride formed by neutralizing donepezil hydrochloride by mixing and stirring the donepezil hydrochloride in a solvent together with an inorganic base comprising a metal such as sodium hydroxide. As a result, a drug-containing solution comprising a metal chloride can be prepared without adding a metal chloride, and by using this drug-containing solution comprising a metal chloride, both the metal chloride and donepezil and the like can be comprised in the final pressure-sensitive adhesive layer. Furthermore, after having formed a metal chloride by mixing and stirring donepezil hydrochloride in a solvent together with an inorganic base comprising a metal, a metal chloride may be further added to the resulting drug (donepezil)-containing solution. Furthermore, examples of inorganic bases comprising a metal include inorganic bases of alkaline metals or alkaline earth metals such as sodium hydroxide, potassium hydroxide, calcium hydroxide, magnesium hydroxide, sodium hydrogen carbonate, potassium hydrogen carbonate, sodium carbonate or potassium carbonate, and from the viewpoint of reducing the likelihood of the formation of by-products, hydroxides of alkaline metals or alkaline earth metals are preferable, sodium hydroxide, calcium hydroxide and magnesium hydroxide are more preferable, and sodium hydroxide is particularly preferable.

In the percutaneously absorbable preparation of the present invention, additives such as antioxidants, various types of pigments, various types of fillers, stabilizers, drug dissolution assistants or drug dissolution inhibitors can be incorporated in the pressure-sensitive adhesive layer as necessary. For example, although there are no particular limitations on such stabilizers or antioxidants, 2-mercaptobenzimidazole, ascorbic acid, ascorbyl palmitate, sodium metabisulfite, sodium sulfite and sodium bisulfite are preferable, and one type or a combination of two or more types can be used. The content of the stabilizer or antioxidant in the pressure-sensitive adhesive layer is preferably about 0.0005 to 5 wt%, more preferably 0.001 to 3 wt% and most preferably 0.01 to 1 wt% based on the total weight of the pressure-sensitive adhesive layer.

In the percutaneously absorbable preparation of the present invention, the thickness of the pressure-sensitive adhesive layer is preferably 20 to 300 µm, more preferably 30 to 300 µm and most preferably 50 to 300 µm in order to demonstrate the preferable plasma concentration profile for donepezil and the like of the present invention. Specifically, the percutaneously absorbable preparation of the present invention is preferably a percutaneously absorbable preparation having a pressure-sensitive adhesive layer having a thickness of 20 to 300 µm and which can be administered to a patient so as to demonstrate the preferable plasma concentration profile for donepezil and the like in the present invention. Since AUC is affected by the amount of donepezil and the like in the pressure-sensitive adhesive per unit surface area, it is beneficial to consider the thickness of the pressure-sensitive adhesive layer when adjusting the AUC.

If the thickness of the pressure-sensitive adhesive layer is less than 20 µm, there is the risk of it being difficult to obtain adequate adhesiveness, comprise an effective amount of donepezil and the like and comprise secondary particles of the metal chloride, while if the thickness of the pressure-sensitive adhesive layer exceeds 300 µm, there is the risk of difficulty in coating.

The percutaneously absorbable preparation of the present invention normally comprises a support, a pressure-sensitive adhesive layer, and a release sheet. Namely, the percutaneously absorbable preparation of the present invention has a structure wherein the pressure-sensitive adhesive layer described above is laminated on at least one side of the support, and the adhesive surface of the pressure-sensitive adhesive layer (the surface opposite the side on which the pressure-sensitive adhesive layer is laminated on the support) is preferably protected by being covered with a release sheet until immediately before use. In addition, the preparation may also be in the form of a roll without using a release sheet by coating a silicone, fluorine or wax backing agent and the like onto the support.

Although there are no particular limitations on the support, that in which the content of donepezil and the like in the pressure-sensitive adhesive layer does not decrease as a result of being lost from the back of the support by passing there through (namely, a material impermeable to donepezil and the like) is preferable, and as will be described below, in the case of an aspect in which a liquid plasticizer is compriseed in the pressure-sensitive adhesive layer, a support in which the proportions of donepezil and the like and liquid plasticizer do not decrease as a result of being lost from the back of the support by passing there through (namely, a material impermeable to the liquid plasticizer and the donepezil and the like) is preferable.

Specific examples include polyester (such as polyethylene terephthalate (PET)), nylon, polyvinyl acetate, polyethylene, polypropylene, ethylene-vinyl acetate copolymer, polytetrafluoroethylene, ionomer resins and other single films, metal foil, and laminate films obtained by laminating two or more such films. Among these, the support is preferably that in which a laminate film is obtained by laminating a nonporous film consisting of one of the aforementioned materials with a porous film as described below in order to improve the adhesiveness (anchoring properties) of the support with the pressure-sensitive adhesive layer, and the pressure-sensitive adhesive layer is formed on the side of the porous film.

There are no particular limitations on the porous film provided it improves anchoring properties with the pressure-sensitive adhesive layer, and examples include paper, woven fabrics, nonwoven fabrics (such as polyester (including polyethylene terephthalate (PET)) nonwoven fabric)), and film- obtained by mechanical perforation of the above-mentioned films (such as polyester, nylon, Saran, polyethylene, polypropylene, ethylene-vinyl acetate copolymer, polyvinyl chloride, ethylene-ethyl acrylate copolymer, polytetrafluoroethylene, metal foil, polyethylene terephthalate and other single films and laminate films obtained by laminating one or two or more such films), while paper, woven fabrics and nonwoven fabrics (such as polyester nonwoven fabric or polyethylene terephthalate nonwoven fabric) are preferable from the viewpoint of flexibility of the support. In consideration of improvement of anchoring properties and flexibility of the pressure-sensitive adhesive layer, the thickness of the porous film is normally 10 to 500 µm, and in the case of a thin percutaneously absorbable preparation such as a plaster or adhesive tape, the thickness is normally about 1 to 200 µm. In the case of woven fabrics or nonwoven fabrics, the basis weight is preferably 5 to 30 g/m² in terms of improving anchoring properties.

There are no particular limitations on the thickness of the support for the percutaneously absorbable preparation of the present invention, and is preferably 2 to 200 µm and more preferably 10 to 50 µm. If the thickness of the support is less than 2 µm, handling ease such as self-supporting properties tend to decrease, while if the thickness exceeds 200 µm, the support may feel unpleasant (stiff) and compliance with the skin tends to decrease.

There are no particular limitations on the release sheet, and a known release sheet can be used. Specific examples of the release sheet include a release sheet in which a release agent layer composed of a release agent is formed on the surface of a release sheet base, a plastic film which itself has good release properties, and a release sheet of a configuration in which a release layer, consisting of the plastic film material with good release properties, is formed on the surface of a release sheet base. The release surface of the release sheet may be on only one side of the base or on both sides.

There are no particular limitations on the release agent in this release sheet, examples of which include long-chain alkyl group-containing polymers, silicone polymers (silicon release agents), fluorine polymers (fluorine release agents) and other release agents. Examples of the base for the release sheet include polyethylene terephthalate (PET) film, polyimide film, polypropylene film, polyethylene film, polycarbonate film, polyester (other than PET) film or other plastic film; metal-deposited plastic films obtained by depositing a metal on one of these films; Japanese paper, regular paper, kraft paper, glassine paper, fine paper other paper; nonwoven fabric, fabric or other fibrous material; and metal foil.

In addition, examples of plastic films that themselves have good release properties include polyethylene (such a low-density polyethylene or linear low-density polyethylene), polypropylene, ethylene-propylene copolymer and other ethylene-α-olefin copolymers (block copolymers or random copolymers) as well as polyolefin films formed from polyolefin resins consisting of mixtures thereof, and Teflon (registered trademark) films.

Furthermore, the release layer formed on the surface of the above-mentioned release sheet base can be formed by laminating or coating the material of the above-mentioned plastic film with good release properties on the above-mentioned release sheet base.

There are no particular limitations on the thickness (total thickness) of the release sheet, and is normally 200 µm or less and preferably 25 to 100 µm.

There are no particular limitations on the production method of the percutaneously absorbable preparation of the present invention, and is preferably produced according to, for example, the following production methods of (i) to (iii).
(i) A drug-containing pressure-sensitive adhesive liquid is prepared in which donepezil and/or donepezil hydrochloride is dissolved or dispersed in a solvent together with a pressure-sensitive adhesive and, as necessary, a liquid plasticizer or other additive and the like, and this liquid is then mixed and stirred with a metal chloride or a dispersion of a metal chloride in a solvent such as ethanol followed by the addition of a crosslinking agent. The mixture obtained in this manner is then coated onto one side of a support or a release-treated side of a release sheet followed by drying to form a pressure-sensitive adhesive layer, and after laminating the release sheet or support onto the pressure-sensitive adhesive layer, aging treatment is carried out to crosslink the pressure-sensitive adhesive layer.
(ii) In the case of using a liquid plasticizer, the donepezil and/or donepezil hydrochloride, pressure-sensitive adhesive, liquid plasticizer, and additive used as necessary, are mixed and stirred directly, a metal chloride or dispersion obtained by dispersing a metal chloride in a solvent such as ethanol are mixed and stirred therewith, and a mixture obtained by further adding a crosslinking agent thereto is coated onto one side of a support or on the release-treated side of a release sheet followed by drying to form a pressure-sensitive adhesive layer, and after laminating the release sheet or support onto the pressure-sensitive adhesive layer, aging treatment is carried out to crosslink the pressure-sensitive adhesive layer.
(iii) A drug-containing solution is prepared by mixing and stirring a dispersion obtained by dispersing a metal chloride in a solvent such as ethanol with donepezil and/or donepezil hydrochloride. In the case of using donepezil hydrochloride for the drug, a metal chloride is formed by mixing and stirring the donepezil hydrochloride, a metal hydroxide and, as necessary, a solvent such as ethanol, and neutralizing, followed by further adding and mixing a metal chloride as necessary to prepare a drug-containing solution. On the other hand, a pressure-sensitive adhesive-containing liquid is prepared by dissolving and/or dispersing a pressure-sensitive adhesive in a solvent along with a liquid plasticizer or other additive as necessary, or in the case of using a liquid plasticizer, a pressure-sensitive adhesive-containing liquid is prepared by directly mixing and stirring a pressure-sensitive adhesive, liquid plasticizer, and an additive used as necessary. Subsequently, the above-mentioned drug-containing solution is added to the pressure-sensitive adhesive-containing liquid followed by stirring, further adding a crosslinking agent, applying the resulting mixture to one side of a support or on the release-treated side of a release sheet and drying to form a pressure-sensitive adhesive layer, and after laminating the release sheet or support onto the pressure-sensitive adhesive layer, aging treatment is carried out to crosslink the pressure-sensitive adhesive layer.

In the methods described in (i) to (iii) above, examples of the solvent used to dissolve and/or disperse the pressure-sensitive adhesive and the like include ethyl acetate, toluene, hexane, 2-propanol, methanol, ethanol and water. In addition, these can also be used to adjust viscosity after adding a crosslinking agent.

Furthermore, in the method of (iii), since a preparation can be produced in which the drug ultimately compriseed in the pressure-sensitive adhesive layer mainly comprises donepezil (free form) having superior percutaneous absorbability, a preparation having superior percutaneous absorbability can be reliably produced that can be administered so as to demonstrate the preferable plasma concentration profile for donepezil and the like in the present invention.

In the present invention, the Tmax, Cmax and AUC each vary depending on the concentration of donepezil and the like in the pressure-sensitive adhesive layer, the thickness of the pressure-sensitive adhesive layer, the type of pressure-sensitive adhesive and the type of liquid plasticizer and concentration thereof in the pressure-sensitive adhesive layer. In the percutaneously absorbable preparation of the present invention, by suitably changing the concentration of donepezil and the like in the pressure-sensitive adhesive layer, the thickness of the pressure-sensitive adhesive layer, the type of pressure-sensitive adhesive and the type of liquid plasticizer and concentration thereof in the pressure-sensitive adhesive layer, Tmax, Cmax and AUC can each be made to be within their respective preferable ranges of the present invention.

Thus, an example of a formula of a percutaneously absorbable preparation of donepezil and the like having high commercial practicality is as follows:
concentration of donepezil and the like in pressure-sensitive adhesive layer: 1 to 30 wt% and preferably 3 to 20 wt%;
thickness of pressure-sensitive adhesive layer: 20 to 300 µm and preferably 60 to 240 µm;
type of pressure-sensitive adhesive: acrylic pressure-sensitive adhesive;
type of liquid plasticizer: fatty acid alkyl ester and preferably an ester of a lower monovalent alcohol having 1 to 4 carbon atoms and a saturated or unsaturated fatty acid having 12 to 16 carbon atoms; and
concentration of liquid plasticizer in pressure-sensitive adhesive layer: 10 to 160 parts by weight, and preferably 40 to 150 parts by weight, based on 100 parts by weight of the pressure-sensitive adhesive.

There are no particular limitations on the form of the percutaneously absorbable preparation of the present invention, examples of which include tapes and sheets. In addition, although varying according to the pressure-sensitive adhesive used, the type and amount of liquid plasticizer as well as patient age, body weight, symptoms and the like, the dosage of the percutaneously absorbable preparation of the present invention is preferably such that, in the case of an adult, a percutaneously absorbable preparation comprising 2 to 250 mg of donepezil or donepezil hydrochloride is normally adhered to a 5 to 150 cm² patch of skin once every seven days to once a day in order to demonstrate the preferable plasma concentration profile for donepezil and the like in the present invention. Specifically, the percutaneously absorbable preparation of the present invention is preferably a percutaneously absorbable preparation comprising 2 to 250 mg of donepezil or donepezil hydrochloride that can be administered to a patient so as to demonstrate the preferable plasma concentration profile for donepezil and the like in the present invention. In addition, the percutaneously absorbable preparation of the present invention is preferably a percutaneously absorbable preparation that is applied to 5 to 150 cm² patch of patient's skin that can be administered to a patient so as to demonstrate the preferable plasma concentration profile for donepezil and the like in the present invention. Moreover, the percutaneously absorbable preparation of the present invention is preferably a percutaneously absorbable preparation administered to a patient once every seven days to once a day that can be administered to a patient so as to demonstrate the preferable plasma concentration profile for donepezil and the like in the present invention.

### Examples

Although the following provides a more detailed explanation of the present invention through examples thereof, the present invention is not limited to these examples. Note that "parts" in all of the following descriptions refers to parts by weight.
1. Preparation of Acrylic Pressure-Sensitive Adhesive Solution
   75 parts of 2-ethylhexyl acrylate, 22 parts of N-vinyl-2-pyrrolidone, 3 parts of acrylic acid and 0.2 parts of azobisisobutyronitrile were solution-polymerized in ethyl acetate at 60°C in an inert gas atmosphere to obtain a pressure-sensitive adhesive solution (pressure-sensitive adhesive solid content: 28 wt%).
2. Percutaneously Absorbable Preparation Produced Using Donepezil Hydrochloride

### Example 1

An acrylic pressure-sensitive adhesive solution having a pressure-sensitive adhesive solid content of 40.38 parts and 49.82 parts of isopropyl myristate were mixed and stirred to uniformity in a container, while 8.27 parts of donepezil hydrochloride and an ethanol solution comprising 0.80 parts of sodium hydroxide were mixed and stirred in a separate container. The drug-containing mixture was then added to the mixture of the pressure-sensitive adhesive solution and the isopropyl myristate and the mixture was stirred followed by the sequential addition of 0.05 parts of ascorbic acid, 0.50 parts of sodium metabisulfite and 0.18 parts of ethylacetoacetate aluminum diisopropylate and stirring, adjusting the viscosity with ethyl acetate, coating this liquid onto a silicone release-treated PET film (thickness: 75 µm) to a thickness of 150 µm and drying to form a pressure-sensitive adhesive layer. After laminating the non-woven fabric side of a laminate of a PET film (thickness: 2 µm) and PET non-woven fabric (basis weight: 12 g/m²) onto this pressure-sensitive adhesive layer, aging treatment was carried out for 48 hours at 70°C to obtain a percutaneously absorbable preparation.

### Examples 2

A percutaneously absorbable preparation was obtained in the similar manner as Example 1 with the exception of forming the pressure-sensitive adhesive layer by coating to a thickness of 200 µm after drying.

### 3. Single-Dose Administration Test of Percutaneously Absorbable Preparation

Single-dose administration tests were carried out as described below using the percutaneously absorbable preparations of Examples 1 and 2.

### Examples 3 and 4

A single-dose administration test was carried out on healthy adults by dividing into two groups A and B of 12 subjects each.

Each group was orally administered one 5 mg tablet of commercially available Aricept™ along with drinking water during a Period 1. Blood samples were collected over time until 288 hours after administration to measure the levels of donepezil in plasma.

An adequate washout period was provided following completion of the Aricept™ 5 mg tablet administration test.

A donepezil hydrochloride percutaneously absorbable preparation was adhered to the skin of each subject for 1 week during a Period 2. At this time, a single sheet of the percutaneously absorbable preparation of Example 1 (40 cm²) was adhered to the backs of the subjects of group A, while a single sheet of the percutaneously absorbable preparation of Example 2 (40 cm²) was adhered to the backs of the subjects of group B. Following adhesion, blood samples were collected over time until 528 hours after adhesion to measure the levels of donepezil in plasma.

Measurement of the levels of donepezil in plasma was carried out according to the method described below. An internal standard was added to 100 µL of the resulting plasma followed by extraction of donepezil and the internal standard with an organic solvent (extraction solvent). Next, the organic solvent was distilled off. A solution for HPLC injection was added to the resulting residue to dissolve followed by carrying out quantitative analysis by HPLC/MS/MS.

Changes in the concentrations of donepezil in plasma are shown in FIGS. 1 and 2. In addition, pharmacokinetic parameters as calculated by non-compartment analysis (program: WinNonlin Ver. 5.1.1) based on the concentrations of donepezil in plasma in group A and group B are shown in Table 1.

**Table 1**

| Pharmacokinetic Parameters for Donepezil Hydrochloride Single-Dose Administration for 5 mg Tablet, 150 µm Patch and 200 µm Patch | | | | | | | |
|---|---|---|---|---|---|---|---|
| Regimen | | T1/2(hr) | Tlag (hr) | Tmax (hr) | Cmax (ng/mL) | AUClast (ng·hr/mL) | AUCinf (ng·hr/mL) |
| Aricept 5 mg tablet (A) | Mean | 71.25 | 0.50 | 2.96 | 7.15 | 253.40 | 286.95 |
| | SD | 13.85 | 0.21 | 0.75 | 2.90 | 79.25 | 85.56 |
| Aricept 5 mg tablet (B) | Mean | 78.54 | 0.54 | 3.38 | 5.04 | 232.85 | 267.90 |
| | SD | 17.38 | 0.14 | 1.61 | 1.02 | 54.48 | 61.11 |
| Example 1 (A) | Mean | 94.36 | 5.33 | 70.83 | 5.80 | 1038.87 | 1084.51 |
| | SD | 39.78 | 3.65 | 21.28 | 3.27 | 614.36 | 613.06 |
| Example 2 (B) | Mean | 80.73 | 5.33 | 114.00 | 6.69 | 1331,20 | 1362.86 |
| | SD | 15.02 | 3.45 | 43.57 | 4.06 | 648.39 | 647.21 |

Based on the results of Table 1 and FIGS. 1 and 2, the percutaneously absorbable preparations of the present invention can be expected to inhibit the risk of adverse side effects since plasma donepezil concentrations did not demonstrate a spike within 2 to 5 hours after administration.

In addition, the donepezil-containing percutaneously absorbable preparations of the present invention are preparations that demonstrate preferable pharmacokinetic parameters with respect to Cmax, AUC and Tmax and the like, and are able to be provided in the form of preparations capable of adhering to the skin for long periods of time.

The present application is based on Japanese Priority Patent Application 2008-143591, filed on May 30, 2008 and U.S. Provisional Patent Application 61/129010, filed on May 30, 2008, the entire contents of which are hereby incorporated by reference.

## Claims

1. A percutaneously absorbable preparation comprising 2-[(1-benzylpiperidin-4-yl) methyl]-5,6-dimethoxyindan-1-one and/or a hydrochloride thereof,
wherein the percutaneously absorbable preparation is able to administer to a patient such that Cmax per unit surface area of the percutaneously absorbable preparation is 0.025 to 0.5 ng/ml·cm² in a plasma concentration profile.

2. The percutaneously absorbable preparation according to claim 1, wherein the Cmax is 0.025 to 0.45 ng/ml.cm².

3. The percutaneously absorbable preparation according to claim 1, wherein the Cmax is 0.05 to 0.4 ng/ml·cm².

4. The percutaneously absorbable preparation according to any of claims 1 to 3, wherein AUC per unit surface area of the percutaneously absorbable preparation is 7.5 to 75 ng·hr/ml·cm².

5. The percutaneously absorbable preparation according to claim 4, wherein the AUC is 10 to 62.5 ng·hr/ml·cm².

6. The percutaneously absorbable preparation according to claim 4, wherein the AUC is 12.5 to 50 ng·hr/ml·cm².

7. The percutaneously absorbable preparation according to any of claims 1 to 3, wherein Tmax is 12 to 192 hr.

8. The percutaneously absorbable preparation according to claim 7, wherein Tmax is 24 to 180 hr.

9. The percutaneously absorbable preparation according to claim 7, wherein the Tmax is 48 to 168 hr.

10. The percutaneously absorbable preparation according to any of claims 1 to 3, wherein AUC per unit surface area of the percutaneously absorbable preparation is 7.5 to 75 ng·hr/ml·cm², and Tmax is 12 to 192 hr.

11. The percutaneously absorbable preparation according to claim 10, wherein the Tmax is 24 to 180 hr.

12. The percutaneously absorbable preparation according to claim 10, wherein the Tmax is 48 to 168 hr.

13. The percutaneously absorbable preparation according to any of claims 1 to 3, wherein AUC per unit surface area of the percutaneously absorbable preparation is 10 to 62.5 ng·hr/ml·cm², and Tmax is 12 to 192 hr.

14. The percutaneously absorbable preparation according to claim 13, wherein the Tmax is 24 to 180 hr.

15. The percutaneously absorbable preparation according to claim 13, wherein the Tmax is 48 to 168 hr.

16. The percutaneously absorbable preparation according to any of claims 1 to 3, wherein AUC per unit surface area of the percutaneously absorbable preparation is 12.5 to 50 ng·hr/ml·cm², and Tmax is 12 to 192 hr.

17. The percutaneously absorbable preparation according to claim 16, wherein the Tmax is 24 to 180 hr.

18. The percutaneously absorbable preparation according to claim 16, wherein the Tmax is 48 to 168 hr.

19. A percutaneously absorbable preparation comprising 2-[(1-benzylpiperidin-4-yl) methyl]-5,6-dimethoxyindan-1-one and/or a hydrochloride thereof,
wherein the percutaneously absorbable preparation js able to administer to a patient such that AUC per unit surface area of the percutaneously absorbable preparation is 7.5 to 75 ng·hr/ml·cm² in a plasma concentration profile.

20. The percutaneously absorbable preparation according to claim 19, wherein the AUC is 10 to 62.5 ng·hr/ml·cm².

21. The percutaneously absorbable preparation according to claim 19, wherein the AUC is 12.5 to 50 ng·hr/ml·cm².

22. The percutaneously absorbable preparation according to any of claims 19 to 21, wherein Tmax is 12 to 192 hr.

23. The percutaneously absorbable preparation according to claim 22, wherein the Tmax is 24 to 180 hr.

24. The percutaneously absorbable preparation according to claim 22, wherein the Tmax is 48 to 168 hr.

25. A percutaneously absorbable preparation comprising 2-[(1-benzylpiperidin-4-yl) methyl]-5,6-dimethoxyindan-1-one and/or a hydrochloride thereof,
wherein the percutaneously absorbable preparation is able to administer to a patient such that Tmax of the plasma concentration profile is 12 to 192 hr.

26. The percutaneously absorbable preparation according to claim 25, wherein the Tmax is 24 to 180 hr.

27. The percutaneously absorbable preparation according to claim 25, wherein the Tmax is 48 to 168 hr.

28. The percutaneously absorbable preparation according to claim 1, which is applied to 5 to 150 cm² of a skin of the patient.

29. The percutaneously absorbable preparation according to claim 1, which is applied to the patient one to seven times per week.
